# EUROPEAN PATENT SPECIFICATION

(11) **EP 2 506 815 B1**
(45) Date of publication and mention of the grant of the patent: **24.07.2019**
(21) Application number: 09774755.4
(22) Date of filing: 03.12.2009
(51) Int. Cl.: A61F 13/02, A61F 13/00

(54) **A WOUND DRESSING, AND METHOD AND PRODUCTION LINE OF PRODUCING THE WOUND DRESSING**
WUNDVERBAND UND VERFAHREN SOWIE PRODUKTIONSANLAGE ZUR HERSTELLUNG DES WUNDVERBANDES
PANSEMENT, ET PROCÉDÉ ET CHAÎNE DE PRODUCTION POUR PRODUIRE LE PANSEMENT

(43) Date of publication of application: 10.10.2012
(73) Proprietor: Pharmaplast SAE, Alexandria 23512 (EG)
(72) Inventor: ATTEIA, Mamdouh H., Alexandria 23512 (EG); FARES, Wassim, Alexandria 23512 (EG)
(74) Representative: Holme Patent A/S
(86) International application number: PCT/IB2009/055482
(87) International publication number: WO 2011/067626

(56) References cited:
- US-A- 4 499 896
- US-A- 6 149 614
- US-A1- 2003 120 229
- US-A1- 2004 133 143
- US-A1- 2006 269 592

## Description

The present invention relates to a wound dressing comprising a film sheet of polyurethane. The polyurethane film sheet having a wound facing side and an opposite side, the wound facing side has a peripheral rim zone provided with an adhesive, the peripheral rim zone encircles a central sheet part free of adhesive on both sides, the wound dressing has a moisture vapor transmission rate of at least 1,200 g/m²/24 hour through the part of the wound dressing material including the adhesive layer at the adhesive-coated rim zone, and a moisture vapor transmission rate at the central sheet part of at least 7, 000 g/m²/24 hour, the adhesive at the peripheral rim zone is protected by a first release liner, and a frame part in form of a detachable second release liner on the opposite side encircles at least a part of the central sheet part at the opposite side.

Conventional adhesive wound dressings contain a central pad area surrounded by adhesive for adhering the wound dressing around the wound. Typically a gauze or sponge pad is secured to the adhesive surface in a central location thereby serving as the wound covering material. A release liner is placed over the adhesive area to be removed before application of the wound dressing on the wound.

Normal human skin releases about 500 g/m²/24 hours of water in a resting state, and a huge disadvantage of adhesive articles in medical applications is maceration. If the water released by the skin is unable to evaporate, the prolonged exposure of the skin to moisture results in softening and breaking down of the skin around the wound and healing time becomes substantially extended.

US patent application no. 2003/120229 discloses a method for manufacturing a complex multilayered absorbent wound dressing including a backing layer, an absorbent layer or island pad, and a wound contact layer having a plurality of apertures for allowing passage of wound exudates further into the absorbent layer.

To avoid maceration adhesive polyurethane films, which are breathable, bacterial proof and transparent, have been developed into wound dressings. Breathability allows excess moisture to evaporate thoroughly, preventing maceration of skin surrounding the wound. Typically, known breathable, transparent film dressings have a moisture vapor transmission rate (MVTR) of about 300 g/m²/24hour, which is too low to allow fast healing at minimum scar formation.

The majority of skin wounds heal better when they are kept moist, and the transparent adhesive film dressings allow wound healing in moist environment because they prevent wound drying. Dry scabs or crusts should be avoided because it has been realised that dryness causes new healing cells to burrow deep underneath the scab or crust. Crusts also involve a cosmetic disadvantage in that when the crust is removed a disfiguring depression is left back marking the scar. Moreover, crusts are excellent foods for bacteria and other germs resulting in infection. Thus, for some kinds of wounds, healing the wound may benefit from trapping the natural moisture and natural fluids within the wound enabling new healing cells to develop and cross the wound.

US patent application no. 2006/269592 A1 discloses an adhesive patch for covering a portion of the anatomical surface of a human being, for example a viral lesion. The patch comprises a backing layer and a layer of a skin-friendly adhesive that is substantially free of hydrocolloid particles. The patch adheres to the skin or mucosa, and/or a wound.

However, known breathable and bacterial proof transparent wound dressing films are disadvantageously fully coated with adhesive, which significantly reduces the breathbility and affects water vapor transmission rate of the film at the central area which comes in contact with the wound. Moreover, adhesive presented to the wound sticks to the wound causing trauma at the newly epithelilizing tissues upon removal of the wound dressing.

There is a need for improved wound dressings remedying the shortcomings of known wound dressings.

In a first aspect according to the present invention is provided a wound dressing having a higher breathability than known wound dressings.

In a second aspect according to the present invention is provided a wound dressing allowing wounds to heal faster and with a more cosmetically acceptable result than when using known wound dressings.

In a third aspect according to the present invention is provided a wound dressing that only causes minimum maceration of skin and dryness of wound.

In a fourth aspect according to the present invention is provided a wound dressing that is easier to apply and take off than known film wound dressings.

In a fifth aspect according to the present invention is provided a wound dressing enabling monitoring the healing process without taking off the wound dressing.

In a sixth aspect according to the present invention is provided a wound dressing which can be used when taking a bath.

The novel and unique features whereby this is achieved according to the present invention consists in that the film sheet of polyurethane has a moisture vapor transmission rate at the central sheet part of at least 7,000 g/m²/24 hour. The central sheet part is free of adhesive on both sides, and is the part of the polyurethane film which comes in contact with the wound bed, while the peripheral adhesive-coated rim zone secures the wound dressing to the healthy skin surrounding the wound.

Within the context of the present application the term "Moisture Vapor Transmission Rate (MVTR)" is understood as a measure of the passage of water vapor through a substance, in particular through at least the polyurethane film. Moisture vapor transmission rate is sometimes also named water vapor transmission rate (MVTR). Thus, moisture vapor transmission rate is a measure of breathability, where breathability is measured by the rate at which water vapor passes through, in the units of grams of water vapour per square meter of material per 24 hour. The MVTR values referred to in this application is standard values according to DIN 53122, measured at 38°C and at 90% relative humidity.

In conventional wound dressings of the above kind, the entire area of the wound facing side of a polyurethane film is coated with adhesive. The adhesive coating severely affects and lowers the moisture vapor transmission rate of the adhesive-coated polyurethane material to a level that significantly reduces wound healing time and induces maceration. These drawbacks are effectively remedied by the combined effect of omitting adhesive on a central sheet part of a polyurethane film sheet having a moisture vapor transmission rate of at least 7,000 g/m²/24 hour.

Another advantage is that upon removal of the wound dressing, there is no pain nor trauma because there is no adhesive at the central sheet part at the wound contact side of the dressing.

Yet another advantage is that the wound dressing according to the present invention is chemically safe to use. The non-adherent polyurethane film in contact with the wound bed is chemically inert and is recognised as being non-cytotoxic and non-carcnogenic. No adhesive is in direct contact with the moisture from the wound bed. Adhesive in contact with fluids within a wound bed may slowly dissolve and enter the bloodstream. This risk is eliminated by the absence of an adhesive at the wound-contacting central sheet part. The adhesive-coated peripheral rim zone may in some embodiments according to the present invention have a much higher moisture vapor transmission rate than the corresponsing skin-adhering zone of known polyurethane film wound dressings.

As polyurethane films fully coated with adhesive loose integrity during removal from the skin, wound dressings including such films are difficult or even impossible to remove as one piece from the wound. Soft pliable polymeric polyurethane films of the above high moisture vapor transmission rates are usually very thin and the manufacting methods needs to be adapted for, from these kinds of polyurethane films, making wound dressings maintaining high structural integrity at both the adhesive-free central part and the adhesive peripheral rim zone of the polyurethane film sheet when the wound dressing is to be removed from the wound.

The polyurethane material of the polyurethane film sheet may be selected to have a plurality of different properties that improve user friendliness, contributes to greater comfort for wearers and makes the wound dressing safer to use than known wound dressings. Such properties may include to select a medical grade type of polyurethane to prevent or at least minimize irritation of skin.

Further properties include that the polyurethane film sheet is bacterial proof to avoid penetration of bacteria and subsequent infection of the wound, that the polyurethane film sheet may be impermeable to liquid water enabling the wearer to shower without having to replace the wound dressing after finishing the shower, that the polyurethane film sheet or wound dressing may be stretchable to conform to various curvatures and body movement, e.g. at shoulders, elbows and knees, and that the polyurethane film sheet may be transparent in order to monitor the wound during healing and currently inspect the wound.

The polyurethane film sheet has a peripheral rim zone coated with an adhesive, which peripheral rim zone serves for adhering the wound dressing to the healthy skin around the wound. Since only the limited area of the peripheral rim zone is coated with adhesive, the remaining non-adhering central part of the polyurethane film sheet is available to cover the wound, to thereby have full advantage of the breathability and other physical and chemical properties of the polyurethane material in relation to the wound, as well as non-adherence to wound bed. The adhesive-coated peripheral rim zone is adhered to substantially healthy skin, and the breathability allows for effective evaporation of sweat.

Preferably the area of the central sheet part that is kept free of adhesive can be at least 30% of the total area of a side of the polyurethane film sheet, preferably between 35 - 70% to have a large accessible non-adherent area for wound coverage, and at the same time ensuring that the wound dressing is adequately adhered around the wound and does not gets off at a light touch, friction or other contact. The polyurethane film does not adhere to itself, further ensuring that the central sheet part for covering the wound bed does not attach or stick to itself when applied to a wound.

Suitable adhesives may be or comprise acrylic-based adhesives, hot-melt adhesives or hydrocolloid adhesives.

Within the scope of the present invention the term "acrylic-based adhesive" is to be understood as an adhesive having a content of an acrylic polymer. Acrylic based-adhesives have the advantage of setting rapidly, being very strong due to heavy cross-linking, have high peel and impact strength, tolerates high temperatures and thus have good environmental resistance. Within the scope of the present invention the term "hot-melt adhesive" means a thermoplastic adhesive compound, which usually is solid at room temperature and becomes fluid on heating to be applied on a surface. Within the scope of the present invention the term "hydrocolloid adhesive" means an adhesive that interacts with water in tissue fluid to form a non-adhesive gel.

Exemplary adhesives are e.g. the hypoallergenic adhesive Code: WD2184 obtainable from Everfront Industrial Co., Ltd., P.O. Box 43-435, Taipei, hot melt hypoallergenic adhesive Code: H1540 obtainable from Henkel AG & Co. KGaA, HenkelstraBe 67, 401 91 Dusseldorf, Germany, or the hydrocolloid adhesive products, which contains Super Absorbent Polymer powder made by the applicant.

The adhesives may be provided on e.g. a paper carrier protected by a release liner and subsequently transferred to another media, such as e.g the polyurethane film, by removal of the release liner and contacting the adhesive with the media. The media may thus expediently be transfer coated to the media due to the adhesive having higher affinity to the media than to the paper carrier.

The acrylic-based adhesive may contain povidone-iodine, which is a powerful broad spectrum germicidal agent effective against a wide range of bacteria, viruses, fungi, protozoa, and spores. Other agents serving the same purpose as povidone-iodine may alternatively be present in the adhesive or in the adhesive means. Povidone (polyvinylpyrrolidone, abbrevated PVP) is effective in prevention and treatment of skin infections and treatment of wounds. In addition, iodine has been recognized as an effective broad-spectrum bactericide, and it is also effective against yeasts, molds, fungi, viruses, and protozoans. A suitable povidone-iodine is obtainable as product code PVP-I 30/60 from BASF, Germany.

The thickness of the polyurethane film sheet may be between 5 - 40 µm, preferably between 10 - 30 µm, more preferred between 15 - 25 µm. The most frequently used and preferred polyurethane film sheet has a thickness of 15 µm. These thicknesses are thinner than for other commercially available ready-to use breathable wound dressings having a lower MVTR. The wound dressing manufacturing method may be specifically adapted to take the physical properties of such a thin film of soft elastomeric material into account.

A suitable polyurethane film of 15 µm is obtainable from Epurex Films GmbH & Co.KG, Bayershofer Weg 21, D - 29699 Bomlitz, Germany, as Walotex 2202AC, which is a polyether based extruded polyurethane film with a paper carrier, that in some embodiments can be utilised as a second release liner in the end product wound dressing. A suitable carrier may be any suitable carrier having sufficient structural integrity to support and carry the thin polyurethane film during manufacturing of the wound dressing and can be utilised as the second release liner also when applying the wound dressing on the wound.

It is important to prevent the polyurethane film and the wound dressing from deforming, wrinkling or folding and sticking to itself to avoid that the wound dressing becomes unfit for use. To further ensure that the adhesive cannot stick unintentionally to other surfaces than the intended surface, the side of the polyurethane film sheet, which is coated with adhesive or at a peripheral rim zone, is protected partly or entirely with a first release liner. The first release liner is to be detached immediately before application of the wound dressing around the wound.

Advantageously the first release liner may comprise a first flap and a second flap that are easily removed to expose the adhesive-coated or adhesive-means provided peripheral rim zone before application of the wound dressing on the wound.

Optionally, the first flap and the second flap overlap at least partly, leaving an edge zone of anyone of the first flap or the second flap free to serve as an expedient finger grasping means for at least partly removal of the first release liner.

Since the polyurethane film is selected to be extremely thin to obtain the demanded higher degree of breathability than in known wound dressings, the otherwise flimsy and fragile polyurethane film sheet may need support during application on the wound site. To this aspect the second release liner may advantageously be divided into two separately individually detachable parts, namely a central part and a frame part encircling at least a part of the central sheet part. Once the central part has been removed the flimsy, fragile central non-adhering polyurethane film sheet part is exposed and constitutes a polyurethane film window that can be positioned above the wound. The frame part expediently keeps the polyurethane film window distended. Thus the second release liner or part of the second release liner, advantageously serves as a carrier and a distender means for the polyurethane film sheet in both the manufacturing process and during application of the wound dressing. The second release liner may in a simple embodiment be made out of a paper carrier for the polyurethane film from which the polyurethane film sheet is made.

A suitable stiffness of the frame part may be required enabling the frame part to serve as a handle and reinforcing means facilitating safe manipulation of the wound dressing at no risk that the adhesive-coated edges of the peripheral rim zone come in contact with the wound bed. The frame part may be the only part of the second release liner left on the ready-to-use wound dressing. Both the central part and the frame part are thus detachable from the polyurethane sheet. For example the central part may be detached towards the end of the manufacturing process and the frame part detached when applied to a wound.

Silicon-coated first release liner and/or second release liner may be preferred due to the good non-stick qualities.

A third means of reinforcing the polyurethane film sheet for easy handling during application is casting a polyurethane resin over a polyester scrim lying on a silicon paper carrier to provide a reinforced polyethylene film. The casted polyurethane resin is then conveyed into ovens to dry the polyurethane resin solvent. The resultant is a polyurethane film reinforced with polyester scrim and covered with a silicon paper carrier. The polyester scrim acts as reinforcement means for the polyurethane film without hindering breathability. The polyester scrim is obtainable from Kirson Industrial Reinforcements GmbH, Rossauweg 14, 93333 Neustadt/Donau, Germany, as RPES 4/2 76/80 PVA. A wound dressing including a polyurethane film sheet with polyester reinforced scrim has a MVTR of about 7,000 g/m²/24hour. At the peripheral rim zone, the polyurethane film sheet may be coated with an adhesive having an adhesion power of app. 10 - 12 N/25mm. The coating weight is 40 g/m² of peripheral rim zone giving an MVTR at the adhesive-coated peripheral rim zone of about 3,000 g/m²/24hour.

In a preferred embodiment the polyurethane film sheet may comprise silver or a silver compound. Suitable silver-containing polyurethane films for making the polyurethane film sheet for use in the wound dressing according to the present invention are obtainable from Smith and Nephew Extruded Films Ltd, Gateway Business Park, Broad Lane, Gilberdyke, East Yorkshire, England, as product ID. EU 25 Silver and EU 27 silver, which are polyurethane films with paper carriers. The polyurethane polymer resins of these films are co-extruded with silver containing compounds. Upon contact with wound exudates, silver ions are released, giving antimicrobial activity. In another suitable polyurethane film, which is obtainable from the applicant, the polyurethane resin is casted over a polyethylene silver net lying on a silicon paper carrier. The casted resin is then conveyed into ovens to dry the polyurethane resin solvent. The resultant is a polyurethane film reinforced with polyethylene silver net and covered with a silicon paper carrier. The silver net acts as a means for both reinforcement and for giving antimicrobial activity when the polyurethane film sheet comes in contact with the wound exudates and does not compromis breathability of the composite polyurethane film. The silver net is obtainable from Smith and Nephew Extruded Films Ltd, Gateway Business Park, Broad Lane, Gilberdyke, East Yorkshire, England, as product no. CB21. A wound dressing including a polyurethane film sheet containing silver has an MVTR of 7,000 g/m²/24hour. At the peripheral rim zone, the polyurethane film sheet is coated with an adhesive having an adhesion power of app. 10 - 12 N/25mm. The coating weight is about 40 g/m² of peripheral rim zone giving an MVTR at the adhesive-coated peripheral rim zone of about 1,200 g/m²/24hour.

Another way of incorporating a silver compound in a polyurethane resin is by means of a shear mixer. The mixture of polyurethane resin and silver compound is casted over silicon paper and conveyed into ovens to dry the solvent and crosslink the polyurethane film, thereby obtaining the polyurethane film containing silver. A suitable polyurethane resin is obtainable from Cytec Industries, Anderlecht Str., 33 B-1620 Drogenbos, Belgium, as Ucecoat AB5454. Silver is outsourced from Sinanen-Japan. The name of the grade is Zeomic SAG10NS, which is silver-zeloite complex.

An alternative polyurethane film is made in a similar manner and casted over silicon paper, but instead of the silver compound the polyurethane resin is mixed with cyclodextrin by means of a shear mixer to produce a film able to absorb offensive odors which are always released by infected wounds. Cyclodextrin is outsourced from Wacker-Germany under name of CAVAMAX.

The wound dressing may be manufactured in many different sizes, including 4.5 x 4.5 cm, 4.8 x 6.7 cm, 6 x 7 cm, 10 x 12 cm, 15 x 20 cm, 10 x 25 cm, 20 x 30 cm, 10 x 10 cm, 10 x 15 cm, 10 x 20 cm and 10 x 30 cm. Thus, even though the polyurethane film is selected to be extremely thin, wound dressings having large sizes and high MVTR can be made. The breadth of the peripheral rim zone encircling the polyurethane window may vary depending on the size of the wound dressing. An exemplary breadth may be e.g. 10 to 30 mm for most of the above sizes of wound dressings.

A method for manufacturing a wound dressing as described above comprises at least the steps of
(a) providing a width of a first laminate comprising the adhesive,
(b) providing through-openings through the first laminate by means of die cutting with a first die,
(c) delaminating the width of first laminate to expose the adhesive,
(d) providing a width of a second laminate comprising a polyurethane film having a moisture vapor transmission rate of at least 7,000 g/m²/24 hour, which polyurethane film is supported by a carrier,
(e) producing a third laminate of wound dressing material by laminating the width of second laminate to the delaminated width of first laminate having through-openings to apply the adhesive to the polyurethane film to thereby obtain a moisture vapor transmission rate of at least 1,200 g/m²/24 hour through the part of the wound dressing material including the adhesive layer, and
(f) from the third laminate of wound dressing material providing a wound dressing with a peripheral rim zone provided with an adhesive, which peripheral rim zone encircles a central polyurethane sheet part free of adhesive on both sides,
   wherein the wound dressing is made by means of die cutting with a second die having a larger outline than the first die.

Within the scope of the present invention the term "laminate" is to be understood as a material constructed by uniting two or more layers of material together by heat sealing and/or pressure application. The process of creating a laminate is lamination.

The first laminate and the second laminate can be obtained as intermediate goods and stored as stock goods on large rolls to be unwinded at suitable speed for the manufacturing process, or be manufactured in the production line and used directly in the method according to the present invention.

The through-openings made in the first laminate are intended for making wound inspection windows covered with a polyurethane film in step (e). Subsequent to making the through-openings, e.g. by die cutting, the first laminate is delaminated, and the adhesive is exposed enabling the holed, delaminated first laminate to be laminated to a second laminate comprising a polyurethane film having a moisture vapor transmission rate of at least 7,000 g/m²/24hour, to produce a third laminate of wound dressing material. The polyurethane film is supported by a carrier, e.g. a silicon-coated paper carrier, providing integrety and rigidity to both the polyurethane film during the lamination process, and to the third laminate. The moisture vapor transmission rate through the part of the third laminate containing the adhesive layer is at least 1,200 g/m²/24hour, which allows moisture, such as sweat, to evaporate during the period the wound dressing is attached to the healthy skin around the wound. Moreover, the inventive wound dressing maintains intact when the wound dressing is removed.

In step (f) wound dressings are continouosly manufactured of the width of third laminate of wound dressing material, which wound dressings feature an adhesive peripheral rim zone encircling a central polyurethane sheet part, free of adhesive on both sides and serving as a convenient wound inspection window. The final wound dressing does not stick to the wound but only to the healthy skin and allows water vapour to escape in a controlled manner to a degree that promote fast healing.

The first laminate confers skin-adhering properties to the wound dressing. The adhesive may expediently be sandwiched between a third release liner and a fourth release liner, and optionally any or both of said third and fourth release liners can be paper carriers. The third release liner and the fourth release liner protect the adhesive during the initial steps of the manufacturing process and makes it possible to make through-openings with a clear cutting edge, and without the adhesive sticks inadvertently to other surfaces than intended, e.g. to parts of manufacturing tools in the product line or other components.

In a preferred embodiment of a first laminate the third release liner has a higher release force to the adhesive of the first laminate than the fourth release liner. Within the present application the third release liner may be named "a tight release force liner" and the fourth release liner named "a low release force liner" to distinguish between degrees of release force of said two liners. The third release liner is utilised as a convenient backing layer, e.g a paper backing layer, when making the through-openings.

The through-openings through the first laminate is made by means of die cutting with a first die. The process of die cutting will be known to the person skilled in the art of cutting out labels or blanks or similar flat objects from rolls or spools. Both rotary die cutters and flat bed die cutters can be used. Although rotary die cutters are more expensive than flat bed die cutters they are faster and for large productions rotary die cutters may be preferred. Combinations of die cutters can also be used. The presence of a third and fourth release liners on the first laminate prevents frayed cutting edges.

The method may comprise the substep (f1) to kiss cut the polyurethane film carrier of the third laminate to separate out a window part of said carrier above the through-opening to expose a polyurethane film window above at least a part of the through-opening. The kiss cut die punches a parting line in the carrier between the window part and a frame part. The window part may be removed in a further substep (f2) subsequent to step (f1). The remaining frame part may be of a size and outline that corresponds to the through-opening and carries the polyurethane film. The stiffness and rigidity of the frame part expediently can be utilized to keep the polyurethane window distended during manipulation and application of the wound dressing.

The adhesive intended for adhering to the healthy skin is protected with an easy peelable release liner system, the first release liner. The first release liner may be the third release liner already present on the third laminate or a new release liner specifically designed for easy detachment. Accordingly, subsequent to, prior to or simultaneously with finalising the side of the width of third laminate intended for being the side of the wound dressing facing away from the wound, the opposite skin-contacting side may also be worked. The new release liner may be applied in substep (f3) subsequent to, prior to or simultaneously with substep (f2) or (f1'), delaminating the third laminate by removing the third release liner. Once the third release liner has been removed the adhesive-exposed surface is re-protected in substep (f4) subsequent to substep (f3) by the first release liner, which is particular easy to detach.

Preferably, the first release liner may be first and second release liner flaps, which overlap at least partly. The overlapping part is attached to neither adhesive nor polyurethane and serves as an expedient finger grip means for release of a flap. Once the adhesive side of the wound dressing is at least partly free of the first release liner, application of the central polyurethane sheet window above the wound can be started. The wound dressing conforms to body curvatures and any remaining first release liner flap or second release liner flap can be released during application of the wound dressing.

Final wound dressings is made at the end of step (f) from the continuous width of third laminate by means of die cutting with a second die having a larger outline than the first die. The central part of the polyurethane film sheet is intact and the resultant adhesive pheripheral rim zone and the skin contacting side of the polyurethane film window is protected by the first release liner, optionally first and second release liner flaps. The opposite side may a second release liner, in case that the adhesive is supplied to the polyurethane film as an adhesive coating, and/or the polyurethane film sheet reinforced by incorporation of a reinforcement means. The moisture vapor transmission rate through the combined polyurethane film layer, optionally including a reinforcement means and an adhesive layer may be at least 1,200 g/m²/24hour.

Any of the release liners or the carrier support may advantageously be silicon-coated paper release liners. The adhesive may e.g. be an acrylic-based adhesive, a hot-melt adhesive or a hydrocolloid adhesive to which the adhesive power of silicon is low.

To protect the final wound dressing during storage it is preferred to envelope it in a suitable packing in a packaging step (g), in particular since one side of the polyurethane film sheet of the wound dressing always is exposed. One or more wound dressings may be included in one package.

Furthermore, it is preferred for some sterile uses that the final wound dressing is sterilised in a sterilising step (h) . Sterilising has the further advantage of prolonging lifetime in addition to conferring versatile usability to the wound dressing. Typically sterilising is made after packaging, but within the scope of the present invention sterilising and packaging can be made in any order.

The sterilising step (h) may be performed by ethylene oxide gas sterilising, but if the adhesive comprises povidone-iodine the wound dressing is best sterilised by gamma irradiation to avoid iodine sublimation.

A wound dressing having a hitherto unknown high moisture vapor transmission rate is obtained because the through-openings are covered with a very thin polyurethane film having a moisture vapor transmission rate of at least 7,000 g/m²/24hour. Suitable polyurethane films meeting this requirement are as mentioned very thin e.g. between 5 - 40 µm, preferably between 10 - 30 µm, more preferred between 15 - 25 µm and most preferred 15 µm. Within the art of making wound dressings problems of film elongation, rupture, wrinkling and other kinds of deformation have prejudiced the use of such thin polymeric films, in particular polyurethane films having superior MVTR, in particular in large wound dressings. By means of the method use of thin polyurethane films having high MVTR in wound dressing has been made possible.

The polyurethane film included in the wound dressing according to the present invention may be casted over a silver containing net, preferably a polyethylene silver containing net. The silver net reinforces the polyurethane film sheet and confer antimicrobial activity. Upon using the polyethylene silver net reinforced polyurethane film in the production process, the paper carrier of the polyurethane film may be removed prior to die-cutting in step (f). The polyethylene silver net is stiff enough to keep the polyurethane film sheet distended.

Alternatively, during co-extrusion nano silver particles or ionic silver can be added to the solid polyurethane resin in the extruder hopper to produce a polyurethane film containing silver.

The wound dressing obtained using the described method may for example be the wound dressing discussed above, and any features of said wound dressing can be incorporated when manufacturing wound dressings using the above method. The order of the steps of the inventive method may be changed if appropriate. Thus the series of steps in the method as defined above are only intended as a guide and should not be taken to limit the scope of the present invention.

A production line for manufacturing a laminated wound dressing as described above comprises at least
- a first station for unwinding a first laminate comprising an adhesive,
- a second station comprising a die cutter having a first die for providing through-openings through the first laminate,
- a third station for delaminating the first laminate to expose the adhesive,
- a fourth station for providing a width of a second laminate comprising a polyurethane film having a moisture vapor transmission rate of at least 7,000 g/m²/24 hour, which polyurethane film is supported by a carrier,
- a fifth station for laminating the second laminate to the delaminated first laminate to provide a third laminate of wound dressing material having a moisture vapor transmission rate of at least 1,200 g/m²/24 hour through both adhesive layer and polyurethane film, and
- an end cutting station comprising a second die having a larger outline than the first die and is adapted to die cut around the through-openings for separating out the wound dressing from the third laminate.

By using a production line having a plurality of co-operating stations a high production speed can be maintained and the wound dressing be made of e.g. commercially available components. Polyurethane films having high moisture vapor transmission rate are normally very thin which puts demand on the manufacturing process, which need to be a very careful treatment. To coat or apply adhesive directly on a highly elastomeric polyurethane film can be difficult. Such films yield in response to application of the force of the applicator, e.g. a sprayer of a mechanical blade, at the risk of damaging the film window and negatively affecting moisture vapor transmission. By using the lamination and delamination stations the above inconveniences and shortcomings can be substantially avoided.

When the first laminate is combined with the second laminate into the third laminate, the carrier of the second laminate also serves as a carrier for said third laminate. At a kiss-cutting station the carrier of the second laminate is expediently kiss-cut above the through-openings to facilitate removal of a cut out central part of the carrier at the sixth station.

The adhesive of the first laminate may initially be protected by means of a third release liner having strong adherence to the adhesive or, and thus the third laminate. The third release liner serves as a rigid backing when making through-openings in the first laminate in order to section out areas containing adhesive, to make a window for later coverage with polyurethane film. The third release liner is able to engage the cutting tools to make through-openings, but is, due to its tight adhesive force necessary to prevent transfer of adhesive to the fourth release liner when the first laminate is delaminated, not suitable as a first release liner in the final end product wound dressing. However, the third release liner beneficially enforces and carries the adhesive in the production line, and may be kept on the delaminated first laminate, and thus on the third laminate, until the third laminate enters a seventh station where the third release liner can be removed from the side of the third laminate opposite the carrier. The removed third release liner is replaced with a suitable first release liner on the exposed adhesive or adhesive means, preferably a low release force liner. Finally the third laminate constituting the wound dressing material is conveyed to the end cutting station where wound dressings are continuously cut out of the wound dressing material protected with the first release liner.

A sterilising station and/or a packaging station can be arranged at any beneficial location either at the end of the production line or at remote location, whatever is preferred.

In order to achieve a smooth and fast manufacturing process the stations of the production line according to the present invention may include various auxiliary means, machinery and tools for handling and processing feedstock materials and intermediate products.

Auxiliary means may include, but are not limited to, one or more of the following means and installations. For example the first station may comprise a first roller means for supplying the first laminate at a suitable speed.

The second station comprises a die cutter having a first die and the end cutting station comprises a second die having a larger outline than the first die. Thereby the second die is adapted and configured to die cut around the through-openings to delimit an area of polyurethane film covering the through-openings, which area is without adhesive on both side, and to define a polyurethane window for arranging above a wound site.

Furthermore, the third station may comprise a first delaminating roll for winding a fourth release liner off the first laminate, and the fourth station may comprise a second roller means for supplying a second laminate, which at the fifth station may be laminated to the delaminated first laminate by means of lamination means, e.g. pressure rolls with or without supplementary heating.

At the sixth station a second delaminating roll advantageously serves for winding the carrier or kiss cut central parts of the carrier off one side of the third laminate to expose the polyurethane film, and at the same time, prior to or subsequent to unwinding the carrier or kiss cut central parts of the carrier, a third delaminating roll may wind the third release liner off the opposite side of the third laminate at the seventh station to expose the adhesive.

At the release liner station a third roller means may serve for supplying a replacement liner, the first release liner, for covering and protecting the adhesive or adhesive layer of the third laminate. The third roller means may comprise a first flap roll and a second flap roll for providing the first release liner in the form of a first flap and a second flap, which may or may not cover each other at least partly to present finger grip means, optionally also having a back-folded finger grip edge part.

Both the first die cutter and the second die cutter are preferably rotary die cutters although flat bed die cutter also can be used.

The production line may comprise conveyor means for assisting during the continuous supply and transport of first and second feedstock laminates or intermediate products laminates through the production line. The conveyor means of the production line comprises powered nip roller units, e.g. pneumatically powered nip roller units. Nip rolls or pinch rolls are useful for pressing two or more sheets together to form a laminated product. The high pressure created at the nip point brings the sheets or laminates into intimate contact, any bubbles or blisters that might cause a defective bond are squeezed out. Moreover, the nip roller units serve for pulling feedstock laminates supplied to the production line off first and second laminate rolls.

The production line enables manufacturing of wound dressings with very high moisture vapor transmission rate at e.g. high linear speed of about 20m/min. This high speed shall only be taken as exemplary, and the rotational speeds of roller means and nip roller units may be adjusted to manufacture wound dressings both faster and slower if more preferred. Wound dressing in many sizes and compositions can be obtained using the above method and production line by adjusting rotational speed, selecting appropriate dies and suitable laminates.

When the wound dressing is applied to a wound the first release liner is removed to expose the adhesive peripheral rim zone to be adhered around the wound out of contact with the wound bed. The non-adhesive part of the polyurethane film that covers the through-opening of the wound dressing is arranged above the wound with the adhesive facing the skin, and the peripheral adhesive rim zone is then adhered to the healthy skin around the wound for example using the fingertips to put the dressing firmly in place. The frame part of the second release liner is removed, optionally gradually removed, to allow the polyurethane film become resilient and conform to the body anatomy around the wound. Removing the entire frame part of the second release liner at this stage is also possible.

Optionally the polyurethane film sheet, which has been more or less made free of the frame part of the second release liner, is also stretched for optimum fit.

The wound dressing made of a thin polyurethane film sheet has minimum coverage with adhesive, which only is present at a peripheral rim zone. In particular a large polyurethane window is free of adhesive on both sides. Use of the wound dressing according to the present inventions allows for fast healing and continuous monitoring of the healing process and healing progression.

The invention will now be described by way on exemplary embodiments with reference to the accompanying drawing, in which
fig. 1 shows, seen in perspective, an exploded view of a wound dressing according to the present invention,
fig. 2 shows, seen in perspective oblique from the side facing opposite the skin, the wound dressing seen in fig. 1 in assembled state,
fig. 3 shows, seen in perspective, an exploded view of a second embodiment of a wound dressing not part of the present invention,
fig. 4 shows, seen in perspective oblique from the side facing opposite the skin, the wound dressing seen in fig. 3 in assembled state,
fig. 5 shows, seen in perspective, an exploded view of a third embodiment of a wound dressing not part of the present invention,
fig. 6 shows, seen in perspective oblique from the side facing opposite the skin, the wound dressing seen in fig. 5 in assembled state,
fig. 7 shows schematically a first production line for manufacturing the wound dressing according to the present invention,
fig. 8 shows schematically a second production line for manufacturing the wound dressing shown in fig. 3, and
fig. 9 shows schematically a third production line for manufacturing the wound dressing shown in fig. 5.

The wound dressings according to the present invention are described in more detail below by way of exemplary designs of the wound dressing. Although the wound dressings are shown in the figures to be rectangular with smooth curves, the figures should not be construed to limit the design and outline of the wound dressings according to the invention. Many other outlines are intended within the scope of the present invention, including squared, oval, and circular, as well as any suitable polygonal design and outline. The thicknesses of the various layers are indicated by way of example. Although it is an advantage for allowing wound inspection and indicated in the drawing, the polyurethane film need not be transparent.

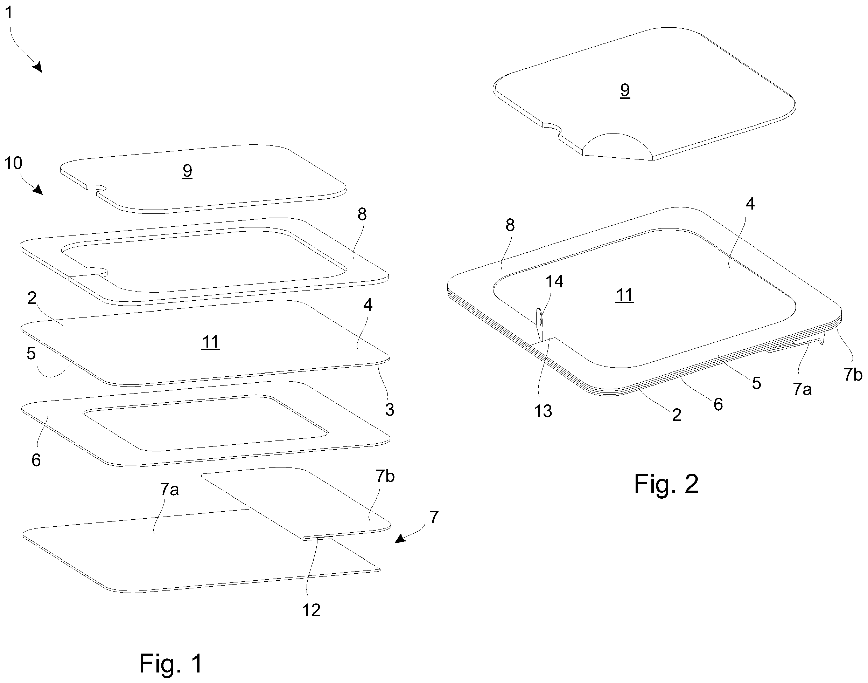

Fig. 1 show an exploded view of an exemplary wound dressing 1 consisting of a transparent polyurethane film sheet 2, having a skin-contacting side 3, and an opposite side 4 facing away from the wearer subsequent to application on a wound. The skin contacting side has a peripheral rim zone 5 coated with an adhesive 6 to facilitate reliable attachment around a wound. The adhesive 6 is protected by a first detachable realise liner 7, in the case shown a first release liner flap 7a overlapping a second release liner flap 7b. The opposite side 4 of the polyurethane film sheet 2 is completely free of adhesive and is protected by an easy detachable second release liner frame 8 made by kiss cutting a central part 9 out of a sheet 10 of second release liner material. The frame part 8 keeps the framed thin polyurethane film window 11 distended prior and during at least partly application of the wound dressing 1 on the wound. The frame part 8 can or cannot, as preferred and convenient, be more or less removed at any appropriate stage of use and application of the wound dressing 1. The frame part 8 encircles the adhesive-free polyurethane window 11 to be arranged just above the wound bed so that the adhesive 6 can stick to substantially healthy skin tissue. Because the polyurethane material is transparent the application process can be monitored, as well as it allows for inspection of wound healing at any time. The second release liner flap 7b has a folding 12 to be grasped when the second release liner flap 7b is removed.

Fig. 2 shows the assembled ready-to-use wound dressing 1 presenting the adhesive-free polyurethane window 11 with the central part 9 fully detached from the sheet 10. The second release liner frame 8 has a parting line 13 merging into a finger flap 14 which is shown detached and folded backwards to illustrate that the second release liner frame 8 is also detachable arranged on the polyurethane film sheet 2. The second release liner frame 8 keeps the polyurethane window 11 distended.

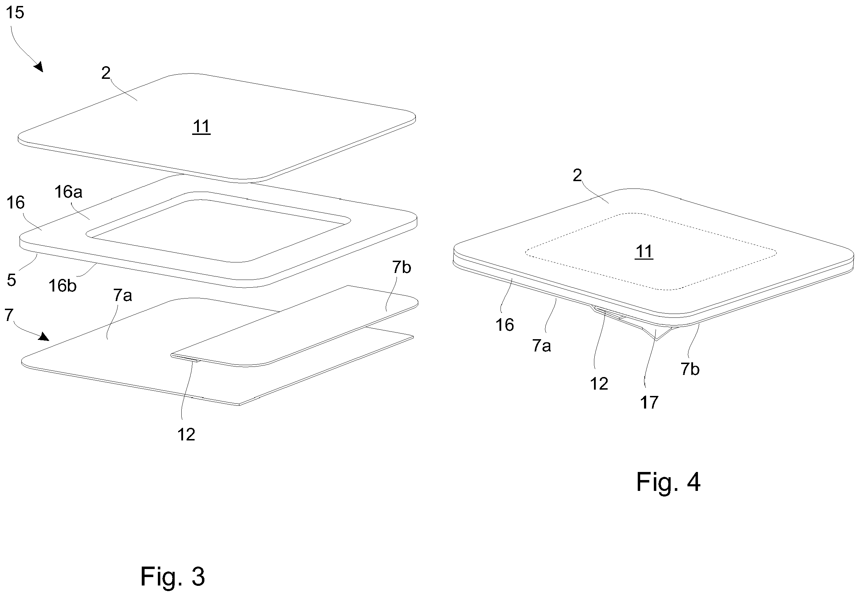

Fig. 3 shows an exploded view of a wound dressing 15 not part of the invention. The wound dressing 15 differs from the wound dressing 1 in that it has no second release liner frame and includes an adhesive means 16 instead of or further to the adhesive coating. The polyurethane film sheet 2 and the first and second release liners flaps 7a,7b are the same and same reference numeral are used. The adhesive means 16 is provided at the skin-contacting side 3 of the polyurethane film sheet 2 as a polyethylen foam frame provided on both sides 16a,16b with suitable adhesives for adhering tight to the polyurethane film sheet 2, and adhering at low release force to the first release liner 7a,7b, respectively. The polyethylene foam frame 16 serves to keep the polyurethane film sheet 2 distended, but is contrary to the second release liner frame part 8 situated on the skin-contacting side of the polyurethane film sheet and faces towards the wound, as the polyethylene foam frame 16 has the double function of also serving for adhering the wound dressing 15 to the skin. The polyethylene foam frame is bendable and conforms easily to any curvature. Moreover, the thickness of the polyethylene foam frame 16 lifts the polyurethane films sheet away from the wound bed. The polyethylene foam frame also serves to absorb sweat and humid that do not evaporate from the skin despite the high MVTR of at least 1,200 g/m²/24hour to further prevent maceration.

Instead of using a polyethylene foam frame a spunlaced non-woven frame may be used as an adhesive means capable of keeping the polyurethane window 11 distended, however any web or foam that are able of conferring the above properties to the wound dressing can be incorporated just as well.

Fig. 4 shows the wound dressing 15 in an assembled state illustrating the transparent polyurethane film sheet 11 overlaying the adhesive polyethylene foam frame 16. A small finger flap 17 is backfolded for easy release of the first release liner flap 7a.

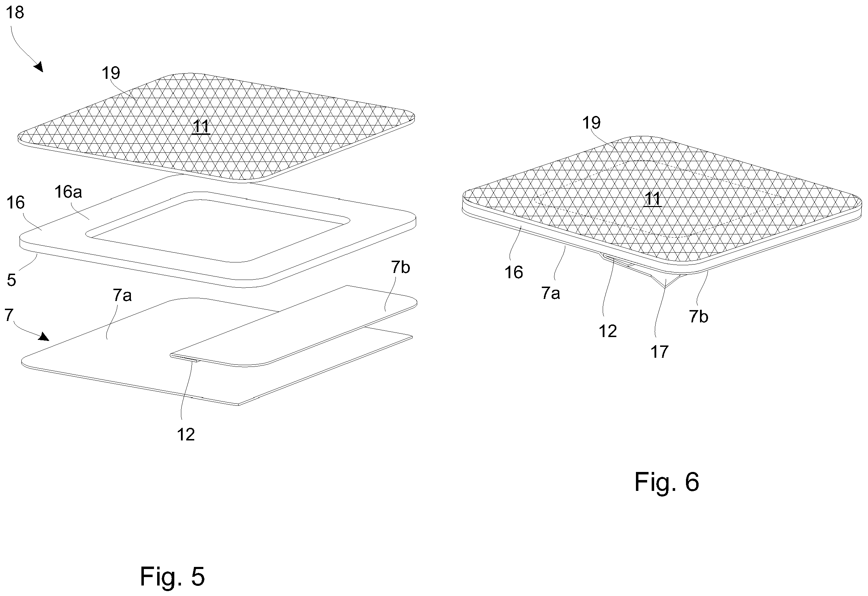

Fig. 5 shows, seen in perspective, an exploded view of a wound dressing 18, not part of the invention. The wound dressing 18 corresponds substantially to the wound dressing 15, and for like parts same reference numerals are used. Fig. 6 shows the same in assembled state.

The wound dressing 18 differs from the wound dressing 15 only in that the polyurethane sheet 2 is replaced with a sheet of polyurethane film casted on a silver net 19 conferring additional integrity, and structural force, and makes the polyurethane film sheet bacteria proof.

The second release liner frame part 8 or the adhesive means frame 16 ensures that the wound dressings 1,15,18 maintain structural integrity during application on the wound, without getting into contact with the polyurethane film sheet 2,19 and contaminate or damage the wound contacting polyurethane window 11. No adhesive or adhesive means is present on any side of the polyurethane sheet window 11.

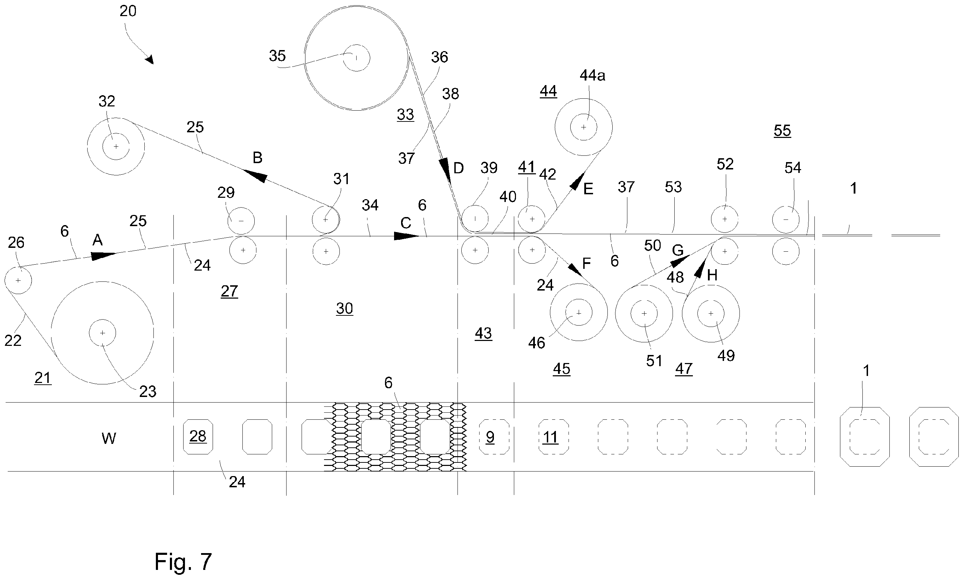

Fig. 7 shows schematically a production line 20 for manufacturing the wound dressing 1.

The production line 20 has a first station 21 for unwinding a width W of first laminate 22 from a first roller means 23, the supply roll 23, as indicated by arrow A. The first laminate 22 comprises the adhesive 6 sandwiched between a width of tight adhesive force third release liner 24 on one side and a width of low force fourth release liner 25 on an opposite side. The first laminate 22 is conveyed via conveyer roll 26 to a second station 27 where through-openings 28 is cut through the first laminate 22 by means of rotational through die cutter 29. The die cut first laminate 22 is, as indicated by arrow B, delaminated at a third station 30 by means of nip rolls 31 and first delaminating roll 32, which winds up the fourth release liner 25 to expose the adhesive 6. The third release liner 24 remains on the delaminated first laminate 34 during the further processing when the width W of the delaminated first laminate 34 leaves the third station 30, as indicated by arrow C. The first laminate 22 has been stripped to expose the adhesive 6 on the width of tight third release liner 24 and the delaminated first laminate 34 is now penetrated by holes, constituting a continuous row of through-openings 28, arising from cutting with rotational through die cutter 29.

The fourth station 33 includes a second roller means 35 for supplying a width of second laminate 36 to the production line 20 at a speed suitable for lamination to the delaminated first laminate 34. The second laminate 36 consists of a width of polyurethane film 37 having a moisture vapor transmission rate of at least 7,000 g/m²/24 hour, and a width of carrier 38, serving as a reinforcing layer for the width of polyurethane film 37. The width of delaminated first laminate 34 is laminated to the polyurethane film 37 of the second laminate 36 in a fifth station 43 by means of pressurised nip rolls 39, which also pulls the second laminate 36 off the second roll means 35, as indicated by arrow D, into intimate uniting contact with the delaminated first laminate 34 to produce a width of third laminate 40.

The third laminate 40 is kiss cut through the carrier 38 at kiss cutter station 41, and the cut out central part 42 of the carrier 38 is subsequently winded on a second delaminating roll 44a at a sixth station 44, as indicated by arrow E, exposing a polyurethane window 11 surrounded by carrier 38. Simultaneously the tight force third release liner 24 is stripped off the third laminate 40 at a seventh station 45 by means of third delaminating roll 46, as indicated by arrow F, exposing the adhesive 6, which now has been transferred or applied to the polyurethane film 37. The moisture vapor transmission rate is at least 1,200 g/m²/24hour through both adhesive 6 and polyurethane film 37.

The width of stripped third laminate 40 is conveyed to a release liner station 47 where, by means of nip rolls 52, as indicated by arrow G, a width of second release liner flaps 50 is applied, to the adhesive 6 by means of first flap roll 49, and a width of first low release liner flaps 48 is applied to the remaining exposed part of the adhesive 6 by means of a second flap roll 51, in overlapping relationship with the width of second low release liner flaps 50. The width of final wound dressing material 53 is conveyed by rotational second die cutter 54 serving to the end cutting station 55. The second die cutter 54 punches around the through-openings 28 with a larger outline than the first die cutter 29 to produce a so-called Frame Style Pattern Coated Transparent Film wound dressing 1.

Since the polyurethane film is selected to be extremely thin to obtain a desired higher degree of breathability than in known wound dressings, the otherwise flimsy polyurethane film sheet may need support during application on the wound. To this aspect, the polyurethane film paper carrier were kiss cut during the manufacturing process into two separately individually detachable parts, namely a central window part and a frame part encircling the central window part. The central window part is removed inline during the production process, leaving a frame part and a transparent polyurethane film sheet window that can be positioned above the wound. During application, the central non-adhesive part of the polyurethane film sheet is positioned over the wound, and the wound dressing is secured to the skin by applying the adhesive peripheral rim zone 5 to the healthy skin surrounding the wound. Finally, the paper carrier frame part 8 is removed and discarded, leaving a fully transparent polyurethane film sheet to cover the wound bed by its adhesive-free central part 11, and well secured to the skin at the peripheral adhesive-coated rim zone 5.

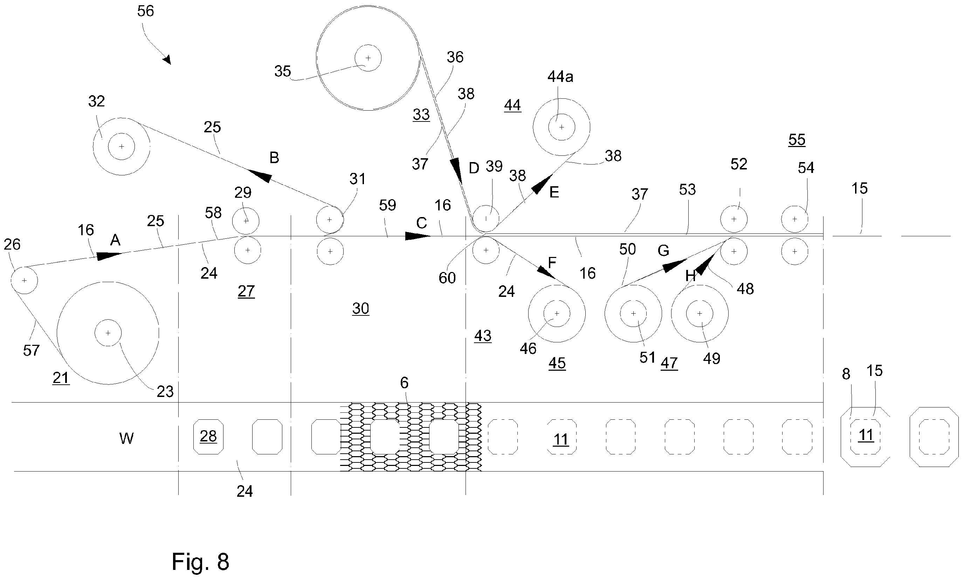

Fig. 8 shows schematically a production line 56 for manufacturing the wound dressing 15. The second production line 56 for the wound dressing 15 corresponds substantially to the production line 20 for the wound dressing 1 and for like parts same reference numerals are used.

The second production line 56 differs in that the first laminate 22 including an adhesive 6 is replaced with an alternative first laminate 57 including an adhesive means 58 protected by a low release force third release liner 24 on the side of the adhesive means 58 intended for being adhered to the skin, and a low release force fourth release liner 25 on the opposite side. The adhesive means 58 may for example be a width of polyethylene foam 58 or a spun-laced non-woven web provided with suitable adhesives on both sides. The alternative first delaminated laminate 59 is not kiss cut since the polyethylene foam or similar adhesive means is self-supported. Consequently the second production line 56 excludes the kiss-cutting station. The entire carrier 38 is, at the sixth station 44, removed from the third laminate 60, which only exists shortly when passing laminating nip rolls 39.

Apart from the above differences all productions station belonging to the second production line function in a similar manner as in the first production line. The polyurethane film carrier, for example a silicon paper carrier, is delaminated before the end die cutting. The final end product wound dressing is an adhesive free polyurethane film, laminated on adhesive-coated polyethylene foam or spun-laced non-woven frame, which is covered with two overlapping silicon paper flaps for easy opening. During application, the two silicon paper flaps are removed to expose the adhesive side of the polyethylene foam or spun-laced non-woven frame, then the adhesive free transparent polyurethane film sheet is positioned on the wound site and the adhesive polyethylene foam or spun-laced non woven frame secures the wound dressing to the healthy skin surrounding the wound.

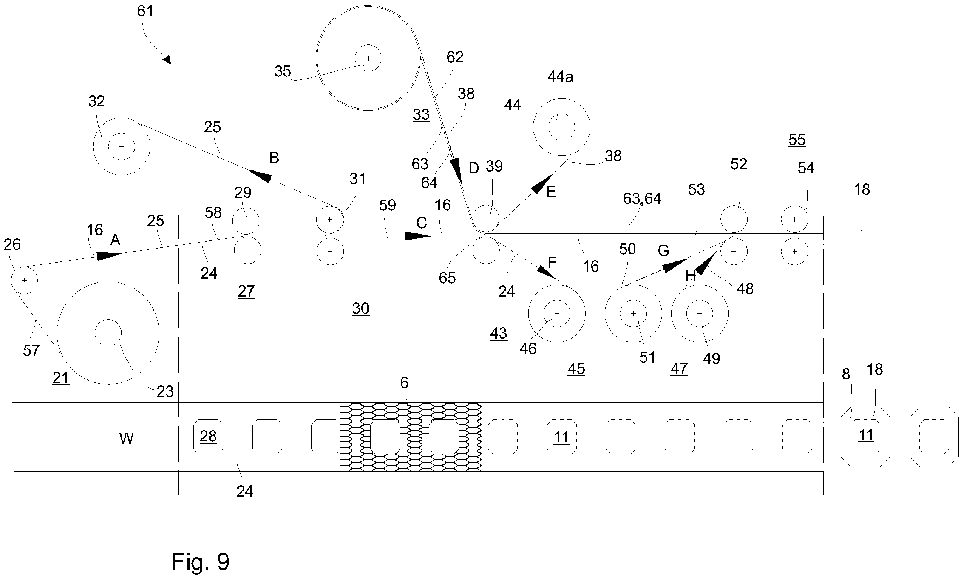

Fig. 9 shows schematically a production line 61 for manufacturing the wound dressing 18. The production line 61 corresponds substantially to the production line 56 and for like parts same reference numerals are used.

The production line 61 differs only in that the second laminate 62 is a composite comprising a polyurethane film 63 casted over a reinforcing silver net 64. The second laminate 62 is combined with the delaminated first laminate 59 to a third laminate 65. Apart from these differences all productions stations belonging to the production line 61 function in a similar manner as in the production line 56.

## Claims

1. A wound dressing (1) comprising
- a polyurethane film sheet (2) having a wound facing side (3) and an opposite side (4),
- the wound facing side (3) has a peripheral rim zone (5) provided with an adhesive (6),
- the peripheral rim zone (5) encircles a central sheet part (11) free of adhesive (6) on both sides (3,4),
- the wound dressing (1) has a moisture vapor transmission rate of at least 1,200 g/m²/24 hour through the part of the wound dressing material including the adhesive layer at the adhesive-coated rim zone (5),
- the adhesive (6) at the peripheral rim zone (5) is protected by a first release liner (7), and
- a frame part (8) in form of a detachable second release liner on the opposite side (4) encircles at least a part of the central sheet part at the opposite side (4),
**characterised in that** the film sheet (2) of polyurethane has a moisture vapor transmission rate at the central sheet part (11) of at least 7,000 g/m²/24 hour.

2. A wound dressing (1) according to claim 1, wherein the polyurethane of the polyurethane film sheet (2) is any of medical grade, bacteria proof, impermeable to liquid water, stretchable, or transparent.

3. A wound dressing (1) according to any of the claims 1 or 2, wherein the area of the central sheet part (11) is between 35 - 70% of the total area of a side of the polyurethane film sheet (2).

4. A wound dressing (1) according to any of the claims 1 - 3, wherein the adhesive (6) comprises an acrylic-based adhesive, a hot-melt adhesive or a hydrocolloid adhesive.

5. A wound dressing (1) according to any of the claims 1 - 4, wherein the adhesive (6) is hypoallergenic.

6. A wound dressing (1) according to any of the claims 1 - 5, wherein the adhesive (6) contains povidone-iodine.

7. A wound dressing (1) according to any of the claims 1 -6, wherein the thickness of the polyurethane film sheet (2) is between 5 - 40 µm, preferably between 10 - 30 µm, more preferably between 15 - 25 µm, and most preferred 15 µm.

8. A wound dressing (1) according to any of the claims 1 - 7, wherein the first release liner (7) comprises a first flap (7a) and a second flap (7b).

9. A wound dressing (1) according to claim 8, wherein the first flap (7a) and the second flap (7b) overlap at least partly.

10. A wound dressing (1) according to any of the claims 1 - 9, wherein any of the first release liner (7) and the second release liner (10) is silicon-coated.

11. A wound dressing (1) according to any of the claims 1 - 10, wherein the polyurethane film sheet (2) comprises silver or a silver compound.

## Patentansprüche

1. Wundverband (1), umfassend
- eine Polyurethan-Folienbahn (2) mit einer wundseitigen Seite (3) und einer gegenüberliegenden Seite (4), wobei
- die wundseitige Seite (3) einen peripheren Randbereiche (5) aufweist, der mit einem Klebstoff (6) versehen ist,
- der periphere Randbereich (5) ein zentrales Blattteil (11) umgibt, das auf beiden Seiten (3, 4) klebstofffrei ist,
- der Wundverband (1) eine Feuchtigkeitsdampfdurchlässigkeitsrate von mindestens 1200 g/m² /24 Stunden durch den Teil des Wundverbandsmaterials einschließlich des klebstoffbeschichteten Randbereiches (5) aufweist,
- der Klebstoff (6) am peripheren Randbereich (5) durch eine erste Trennschicht (7) geschützt ist, und
- ein Rahmenteil (8) in Form einer abnehmbaren zweiten Trennschicht auf der gegenüberliegenden Seite (4) mindestens einen Teil des zentralen Blattteils auf der gegenüberliegenden Seite (4) umgib,
**dadurch gekennzeichnet, dass** die Folienbahn (2) aus Polyurethan eine Feuchtigkeitsdampfdurchlässigkeitsrate im zentralen Blattteil (11) von mindestens 7000 g/m² /24 Stunden aufweist.

2. Wundverband (1) nach Anspruch 1, wobei das Polyurethan der Polyurethan-Folienbahn (2) eines mit medizinischer Reinheit, bakterieller Sicherheit, Undurchlässigkeit für flüssiges Wasser, dehnbar oder transparent ist.

3. Wundverband (1) nach einem der Ansprüche 1 oder 2, wobei die Fläche des zentralen Blattteils (11) zwischen 35 bis 70 % der Gesamtfläche einer Seite der Polyurethan-Folienbahn (2) ist.

4. Wundverband (1) nach einem der Ansprüche 1 - 3, wobei der Klebstoff (6) einen Klebstoff auf Acrylbasis, einen Schmelzkleber oder einen Hydrokolloidkleber umfasst.

5. Wundverband (1) nach einem der Ansprüche 1 - 4, wobei der Klebstoff (6) hypoallergen ist.

6. Wundverband (1) nach einem der Ansprüche 1 - 5, wobei der Klebstoff (6) Povidon-Jod enthält.

7. Wundverband (1) nach einem der Ansprüche 1 - 6, wobei die Dicke der Polyurethan-Folienbahn (2) zwischen 5-40 µm, vorzugsweise zwischen 10 - 30 µm, besonders bevorzugt zwischen 15 - 25 µm, und am bevorzugtesten 15 µm beträgt.

8. Wundverband (1) nach einem der Ansprüche 1 - 7, wobei die erste Trennschicht (7) eine erste Lasche (7a) und eine zweite Lasche (7b) umfasst.

9. Wundverband (1) nach Anspruch 8, wobei die ersten Lasche (7a) und die zweiten Lasche (7b) zumindest teilweise überlappen.

10. Wundverband (1) nach einem der Ansprüche 1 - 9, wobei jede der ersten Trennschicht (7) und der zweiten Trennschicht (10) silikonbeschichtet ist.

11. Wundverband (1) nach einem der Ansprüche 1 - 10, wobei die Polyurethan-Folienbahn (2) Silber oder eine Silberverbindung umfasst.

## Revendications

1. Pansement (1) comprenant:
- une feuille pelliculaire en polyuréthane (2) possédant un côté tourné vers la plaie (3) et un côté opposé (4) ;
- le côté tourné vers la plaie (3) possède une zone marginale périphérique (5) munie d'un adhésif (6) ;
- la zone marginale périphérique (5) encercle une partie centrale (11) de la feuille exempte d'adhésif (6) sur les deux côtés (3, 4) ;
- le pansement (1) possède un taux de transmission de la vapeur d'eau qui s'élève à au moins 1200 g/m²/24 heures à travers la partie de la matière du pansement qui englobe la couche adhésive à la zone marginale (5) enduite d'un adhésif ;
- l'adhésif (6) à la zone marginale périphérique (5) est protégé par un premier revêtement antiadhésif (7) ; et
- un élément (8) faisant office d'encadrement sous la forme d'un second revêtement antiadhésif amovible sur le côté opposé (4) encercle au moins une partie de la partie centrale de la feuille sur le côté opposé (4) ;
**caractérisé en ce que** la feuille pelliculaire (2) de polyuréthane possède un taux de transmission de la vapeur d'eau à la partie centrale de la feuille (11) qui s'élève à au moins 7000 g/m²/24 heures.

2. Pansement (1) selon la revendication 1, dans lequel le polyuréthane de la feuille pelliculaire en polyuréthane (2) représente n'importe quel polyuréthane choisi parmi un polyuréthane de qualité médicale, un polyuréthane qui résiste aux bactéries, un polyuréthane imperméable à l'eau liquide, un polyuréthane extensible ou un polyuréthane transparent.

3. Pansement (1) selon l'une quelconque des revendications 1 ou 2, dans lequel la surface de la partie centrale (11) de la feuille représente entre 35 et 70 % de la surface totale d'un côté de la feuille pelliculaire en polyuréthane (2).

4. Pansement (1) selon l'une quelconque des revendications 1 à 3, dans lequel l'adhésif (6) comprend un adhésif à base d'un composé acrylique, un adhésif thermofusible ou un adhésif du type d'un hydrocolloïde.

5. Pansement (1) selon l'une quelconque des revendications 1 à 4, dans lequel l'adhésif (6) est hypoallergénique.

6. Pansement (1) selon l'une quelconque des revendications 1 à 5, dans lequel l'adhésif (6) contient de la polyvidone iodée.

7. Pansement (1) selon l'une quelconque des revendications 1 à 6, dans lequel l'épaisseur de la feuille pelliculaire en polyuréthane (2) se situe entre 5 et 40 µm, de préférence entre 10 et 30 µm, de manière plus préférée entre 15 et 25 µm, et de manière de loin préférée s'élève à 15 µm.

8. Pansement (1) selon l'une quelconque des revendications 1 à 7, dans lequel le premier revêtement antiadhésif (7) comprend un premier rabat (7a) et un second rabat (7b).

9. Pansement (1) selon la revendication 8, dans lequel le premier rabat (7a) et le second rabat (7b) se chevauchent au moins en partie.

10. Pansement (1) selon l'une quelconque des revendications 1 à 9, dans lequel n'importe quel revêtement antiadhésif choisi parmi le premier revêtement antiadhésif (7) et le second revêtement antiadhésif (10) est enduit de silicone.

11. Pansement (1) selon l'une quelconque des revendications 1 à 10, dans lequel la feuille pelliculaire en polyuréthane (2) comprend de l'argent ou un composé à base d'argent.
